# EUROPÄISCHE PATENTSCHRIFT

(11) **EP 1 876 998 B1**
(45) Veröffentlichungstag und Bekanntmachung des Hinweises auf die Patenterteilung: **25.04.2012**
(21) Anmeldenummer: 06742796.3
(22) Anmeldetag: 04.05.2006
(51) Int. Cl.: A61F 2/00

(54) **VORRICHTUNG ZUR VERMEIDUNG VON INKONTINENZ, INSBESONDERE HARNINKONTINENZ**
DEVICE FOR THE PREVENTION OF INCONTINENCE, ESPECIALLY URINARY INCONTINENCE
DISPOSITIF PERMETTANT D'EVITER L'INCONTINENCE, NOTAMMENT L'INCONTINENCE URINAIRE

(30) Priorität: 04.05.2005 DE 102005021881
(43) Veröffentlichungstag der Anmeldung: 16.01.2008
(73) Patentinhaber: Aesculap AG, 78532 Tuttlingen (DE)
(72) Erfinder: ABELE, Wolfgang, 78532 Tuttlingen/Donau (DE); ODERMATT, Erich, K., CH-8200 Schaffhausen (CH)
(74) Vertreter: Patentanwälte Ruff, Wilhelm, Beier, Dauster & Partner
(86) Internationale Anmeldenummer: PCT/EP2006/004175
(87) Internationale Veröffentlichungsnummer: WO 2006/117214

(56) Entgegenhaltungen:
- DE-A1- 10 013 519
- US-A- 3 789 828
- US-A- 4 634 443

## Beschreibung

Die Erfindung betrifft eine Vorrichtung zur Vermeidung von Inkontinenz, insbesondere Harninkontinenz, mit einem erweiterbaren Ballon, der bei der Implantation im Bereich der Harnröhre zu liegen kommt und die Harnröhre zur Vermeidung von Inkontinenz anheben und/oder zusammendrücken kann.

Es sind zahlreiche Harninkontinenzbänder mit einem erweiterbaren Ballon bekannt. Es wird verwiesen auf DE 43 28 158 A1, die WO 00/18319, die WO 00/66030 und die WO 03/013392 A1. Bei den bekannten Harninkontinenzbändern besteht der Ballon in der Regel aus Silikon. Silikon ist ein Material, das körperverträglich ist, in geeigneter Weise geformt werden kann und beispielsweise durch Verklebung mit Zuleitungen verbunden werden kann. Es hat sich jedoch gezeigt, dass Silikon bei längerer Implantation zu Erosionen der Harnröhre führen kann.

Die US 4,634,443 offenbart ein gastrointestinales Sphinkterimplantat zur Behandlung von Harninkontinenz mit einer mittels eines Fluids dilatierbaren Manschette. Die DE 100 13 519 A1 betrifft eine implantierbare Sphinkterprothese zur Anwendung bei Harninkontinenz mit einer um die Harnröhre legbarer Manschette mit daran angeordnetem Blähkörper, einer Pumpeneinheit sowie einem Fluidreservoir. Aus der US 3,789,828 geht eine Prothese zur Behandlung von Harninkontinenz mit einem mittels eines Fluids, beispielsweise Gels, befüllten Ballon und einem zusätzlich vorgesehenen Inkontinenzband hervor.

Es ist daher Aufgabe der Erfindung, eine Vorrichtung zur Vermeidung von Inkontinenz mit einem erweiterbaren Ballon zu schaffen, bei dem solche Erosionen vermieden werden.

Gegenstand der Erfindung ist eine Vorrichtung zur Vermeidung von Inkontinenz, insbesondere von Harninkontinenz, mit einem erweiterbaren Ballon, der mit mindestens einer Fluidleitung verbunden ist, durch die das Volumen des Ballons einstellbar ist, wobei sich der Ballon im Innern eines porösen Schlauches befindet. Der Gegenstand der Erfindung dient vorwiegend zur Vermeidung der männlichen und weiblichen Harninkontinenz. Der Gegenstand der Erfindung ist auch für die Stuhlinkontinenz einsetzbar.

Aus der WO 01/26581 A1 ist ein Implantat zur Behandlung der Harninkontinenz bekannt, bei dem ein aufweitbarer Ballon in einer rohrförmigen Hülle angeordnet ist. Diese Hülle dient jedoch nur als Einführhilfe bei der Implantation und wird nach Implantieren des Ballons wieder entfernt.

Es wurde gefunden, dass durch die dauernde Umhüllung des Ballons mit dem porösen Schlauch Erosionen der Harnröhre vermieden werden können. Silikon ist ein Material, das im Körper keine Haftverbindungen eingeht, was einerseits auf die Oberflächeneigenschaften des Silikons zurückzuführen ist, andererseits darauf, dass Silikon eine geschlossene Oberfläche besitzt. Demgegenüber kann sich ein poröser Schlauch mit dem Körpergewebe fest verbinden, indem Zellen des Bindesgewebes in die Poren einwachsen und sich mit dem Material des Schlauches fest verbinden. Dadurch wird die Harnröhre mit einem Narbengewebe bedeckt, das durch das poröse Schlauchmaterial verstärkt ist. Relativbewegungen zwischen dem Ballon und der Harnröhre können auf diese Weise nicht mehr zu einem Durchscheuern der Harnröhre führen. Das Schlauchmaterial kann mit dem Ballon verbunden, beispielsweise verklebt sein. Es kann aber auch bevorzugt sein, keine Verbindung zwischen Ballon und Schlauch vorzusehen, so dass auch nach der Implantation eine Relativbewegung zwischen Ballon und Schlauch möglich bleibt, was für das Wohlbefinden des Patienten beim Bewegen förderlich sein kann.

Als poröse Schläuche eignen sich solche, wie sie beispielsweise als Gefäßprothesen bekannt sind. Sie können aus expandiertem Polytetrafluorethylen bestehen oder auch aus Polyurethan, das durch Sprühtechnik aus gelöstem Polyurethan zu einem Schlauch geformt ist.

Besonders geeignet sind textile Schläuche. Es können gewebte Schläuche und insbesondere gewirkte und gestrickte Schläuche zur Anwendung kommen. Diese finden in der Gefäßprothetik vielfach Anwendung. Die Poren müssen mindestens so groß sein, dass sich Bindegewebe in die Poren einlagern kann. Vorzugsweise sind die Poren > 20 µm, insbesondere > 100 µm. Die Poren sind andererseits ausreichend klein, um eine Berührung der Harnröhre durch den Ballon zu vermeiden. Besonders bevorzugt sind poröse Schläuche mit Netzstruktur, bei denen die Poren wesentlich größer sind und vorzugsweise im Bereich von 0,8 bis 3 mm liegen. Außer den Maschenwaren eignen sich als textile Materialien auch Schlauchgeflechte.

Bei einer bevorzugten Ausführungsform der Erfindung ist der poröse Schlauch zumindest im Bereich des Ballons im Durchmesser elastisch aufweitbar. Für diese Ausführungsform eignen sich besonders Gewirke, Gestricke und Geflechte. Mit Vorteil weist der Schlauch im Bereich des Ballons einen größeren Durchmesser auf als in Bereichen außerhalb des Ballons. Dieser größere Durchmesser kann vorgegeben sein oder durch die elastische Aufweitbarkeit erreicht werden. Mit Vorteil ist der Ballon weiterhin innerhalb des Schlauches in dessen Längsrichtung fixiert bzw. befestigt. Durch eine Verengung des Schlauches außerhalb des Bereiches des Ballons kann eine derartige Fixierung erreicht werden.

Bei einer bevorzugten Ausführungsform ist der Schlauch im Bereich des Ballons kleinporiger als außerhalb dieses Bereiches. Durch die Kleinporigkeit wird erreicht, dass eine Berührung des Ballonmaterials mit der Harnröhre vermieden wird. Außerhalb des Bereichs des Ballons besteht dieses Problem nicht. Deshalb kann dort die Maschenweite bzw. die Porenöffnung des Schlauches sehr groß gehalten werden, beispielsweise bis zum 5 mm, wodurch ein schnelles Verwachsen mit dem Bindegewebe erreicht wird, so dass auf besondere Fixierungen des bandförmigen Schlauches an Körperteilen, beispielsweise an Knochen, entbehrlich wird.

Als Material für einen textilen Schlauch eignen sich besonders Polyvinylidendifluorid (PVDF) oder Polypropylen oder ein Gemisch davon, insbesondere in monofiler Form. Für besondere Anwendungen kann der textile Schlauch zumindest im Mittelbereich auch multifile Fäden, insbesondere Velourfäden aufweisen oder aus solchen bestehen. Dies erlaubt einen besonders schnellen und festen Verbund mit dem Bindegewebe. Der Schlauch kann aus Flachmaterial hergestellt sein und eine Längsnaht besitzen. Bevorzugt wird er als nahtloses Schlauchmaterial hergestellt. Um das um den Schlauch entstehende Narbengewebe in seiner Ausformung zu steuern, kann neben der Porengröße auch das Flächengewicht des Schlauches, insbesondere des textilen Schlauches, von 12 bis 120 g/m² angepasst werden. Je dünner der textile Schlauch ist, um so geringer bildet sich das Narbengewebe aus. Aber auch bei einer dickeren Narbenplatte kann der Druck des Ballones durch eine längere Einwirkungszeit das Narbengewebe dehnen.

Die Wandung des Ballons innerhalb des Schlauchs kann überfüttert sein und sich beim Füllen des Ballons mit Fluid entfalten. In der Regel reicht eine elastische Aufweitbarkeit der Ballonwandung aus. Der Ballon und vorzugsweise auch die mindestens eine Zuleitung bestehen deshalb mit Vorteil in an sich bekannter Weise aus Silikon.

Die mindestens eine Leitung verläuft vorzugsweise entlang des Schlauches und ist vorzugsweise bis mindestens zum Schlauchende geführt. Bei einer anderen Ausführungsform ist es möglich, die mindestens eine Leitung zwischen dem Ballon und einem Schlauchende radial aus dem Schlauch durch dessen Wandung herauszuführen. Bei dieser Ausführungsform kann das Leitungsende unabhängig vom Schlauchende platziert werden. Das freie Leitungsende ist mit Vorteil in an sich bekannter Weise mit einem Fluidventil verschlossen, beispielsweise mit einem durchstechbaren Septum. Dieses Ventil wird bei der Implantation an einer von außen zugänglichen Stelle des Körpers fixiert.

Der Ballon hat normalerweise eine längliche Form mit kreisrundem Querschnitt. Wenn erwünscht, kann dem Ballon auch eine besondere Gestalt verliehen werden, so kann der Schlauch und/oder der Ballon in dem Bereich, der bei der Implantation in der Nähe der Harnröhre zu liegen kommt, beispielsweise tailliert sein oder eine U-Form annehmen. Auch die Wandstärke des Ballons kann variiert werden.

Der Schlauch ist als Band ausgebildet, das den Ballon an beiden Seiten überragt. Dies ermöglicht es, die erfindungsgemäße Vorrichtung in üblicher Weise als Harninkontinenzband zu implantieren.

Bei bevorzugten Ausführungsförmen ist der Schlauch mit einem Kleber versehen, insbesondere mit einem biologischen Kleber. Geeignet hierzu sind insbesondere Fibrinogen und Thrombin. Dieser Kleber führt zu einer Vorfixierung im Körper, bis der poröse Schlauch eingewachsen ist.

Weitere Merkmale der Erfindung ergeben sich aus der nachfolgenden Beschreibung von bevorzugten Ausführungsformen der. Erfindung in Verbindung mit den Unteransprüchen und der Zeichnung. Hierbei können die einzelnen Merkmale jeweils für sich alleine oder zu mehreren in einer Ausführungsform der Erfindung verwirklicht sein.

In der Zeichnung zeigen:
- Figur 1 a:: eine Längsansicht einer Ausführungsform mit dem porösen Schlauch,
- Figur 1b:: einen Ballon mit Zuleitung für die Anordnung innerhalb des Schlauches,
- Figur 2:: einen schematischen Längsschnitt des Schlauches, innerhalb dessen der Ballon angeordnet ist,

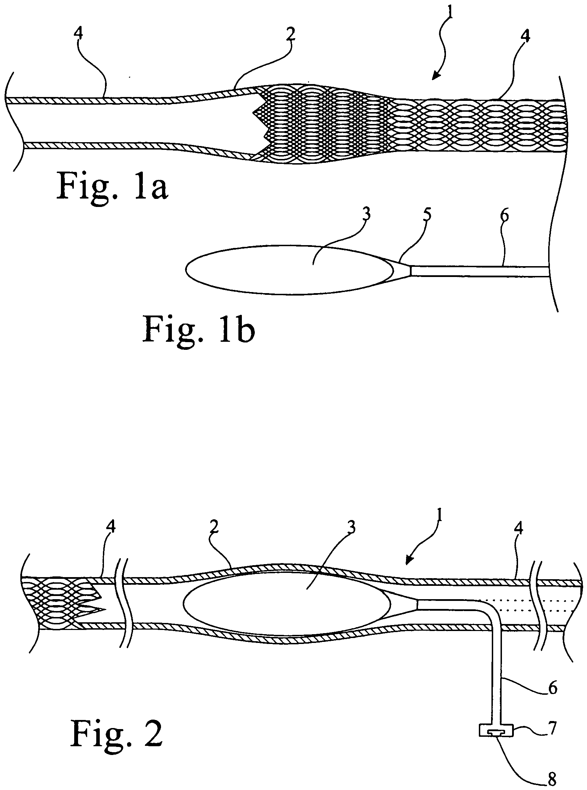

Bei der in der Zeichnung in Figur 1a und b schematisch dargestellten Ausführungsform der Erfindung ist ein gewirkter Schlauch 1 mit einer Länge von ca. 50 cm vorgesehen. Der Schlauch besitzt einen Mittelabschnitt 2, der zur Aufnahme eines Ballons 3 vorgesehen ist. Der Mittelabschnitt hat einen Durchmesser von ca. 25 mm, der ungefähr doppelt so groß ist wie der Durchmesser der sich beidseitig an den Mittelabschnitt 2 anschließenden schlauchförmigen Seitenabschnitte 4. Die Seitenabschnitte 4 besitzen jeweils eine Länge von 20 cm. Der Schlauch 1 ist in Form eines dehnbaren Rundgewirks aus monofilem Polypropylen hergestellt. Das Rundgewirk ist netzförmig und besitzt eine lichte Porenweite von ca. 4 mm in den Seitenabschnitten und von ca. 0,8 mm im Mittelabschnitt 2. Der Mittelabschnitt hat eine Länge von ca. 10 cm.

Der in Figur 1b dargestellte Ballon 3 hat eine Länge von ebenfalls ca. 10 cm und ist ohne die Umhüllung durch den Schlauch bis auf einen maximalen Durchmesser von ca. 40 mm aufweitbar, was einem maximalen Volumen von ca. 125 ml entspricht. Normalerweise reicht ein Volumen von 5 bis 25 ml aus. An einem Ende 5 mündet in den Ballon 3 ein Silikonschlauch 6, der mit dem ebenfalls aus Silikon bestehenden Ballon abdichtend verklebt ist. Ballon und Schlauch können auch aus weichem, elastischem Polyurethan bestehen.

Bei der Ausführungsform nach Figur 2 befindet sich der Ballon 3 bereits im Mittelabschnitt 2 des Schlauches 1. Der Silikonschlauch 6 verläuft zunächst eine gewisse Strecke in einem Seitenabschnitt 4 des Schlauches 1 und ist dann durch die Wandung des Schlauches 1 radial nach außen geführt. Wie durch die gestrichelten Linien erkenntlich, kann der Silikonschlauch 6 auch innerhalb des schlauchförmigen Seitenabschnittes 4 bis an dessen Ende oder darüber hinaus verlaufen. Mit dem freien Ende des Silikonschlauches 6 ist ein Ventil 7 verbunden. Dieses besitzt ein durchstechbares Septum 8. Mit Hilfe einer Spritzennadel kann Fluid, in der Regel eine Flüssigkeit, durch den als Zuleitung dienenden Silikonschlauch in den Ballon eingefüllt werden. Nach Implantation kann der Ballon über das Ventil 7 auf den richtigen Füllgrad eingestellt werden.

Nach Implantation bildet sich zwischen dem Mittelabschnitt 2 des Schlauches 1 und dem die Harnröhre umgebenden Bindegewebe ein Narbengewebe, das fest mit dem Netzschlauch 1 verwächst. Relativbewegungen zwischen dem Ballon 3 aus Silikon und der Innenfläche des Mittelabschnittes 2 des Bandes 1 wirken sich nicht bis zur Harnröhre aus, da das Narbengewebe, das durch das Netzmaterial des Schlauches verstärkt ist, eine mechanische Abschirmung darstellt.

Da das Gewirk aus den monofilen Fäden elastisch dehnbar ist, kann sich der Mittelabschnitt beim Füllen des Ballons 3 bis zu einem gewissen Grade elastisch aufweiten und die Bewegung des Ballons mitmachen. Erreicht die Dehnbarkeit ihren Grenzwert, dann führt ein weiteres Füllen des Ballons zu einem starken Druckanstieg, so dass das Erreichen des maximalen Füllgrades leicht erkennbar ist.

Die verwendeten Materialien, insbesondere das Material des Schlauches und des Ballons sowie der Zuleitung können mit antimikrobiellen Substanzen versehen sein, welche sich in den Materialien und/oder an deren Oberfläche befinden. Dadurch können Infektionen in Folge der Implantation oder auch spätere Infektionen verhindern werden. Weiterhin ist es in an sich bekannter Weise möglich, die verschiedenen Teile des Harninkontinenzbandes mit Markern zu versehen, die mit physikalischen Methoden, wie Röntgenographie oder Ultraschall und dergleichen detektierbar sind. Dadurch kann die korrekte Lage des Harninkontinenzbandes im Körper bei der Implantation und auch später eingestellt und kontrolliert werden.

Das Implantat kann durch eine minimalinvasive Operationsweise analog der transobturator Technik (TOT) bei der weiblichen Harninkontinenz oder durch eine andere minimalinvasive Operationstechnik aus dem Bereich der weiblichen Harninkontinenz implantiert werden. Auch kommen andere alternative Zugangswege von unten, z.B. der Zugang vom Bereich des Perineums oder im Bereich des Schambeines in Frage, um das Implantat minimalinvasiv zu platzieren. Das Implantat kann sowohl über als auch unter der Urethra platziert werden, je nachdem wird auch der minimalinvasive Zugang gewählt. Die Platzierung des Ballons über der Urethra ist bei Harninkontinenz das bevorzugte Implantationsverfahren. Zur Verhinderung von Stuhlinkontinenz wird die Vorrichtung im Bereich des Schließmuskels implantiert.

## Patentansprüche

1. Vorrichtung zur Vermeidung von Inkontinenz, insbesondere von Harninkontinenz, mit einem erweiterbaren Ballon (3), der mit mindestens einer Fluidleitung (6) verbunden ist, durch die das Volumen des Ballons (3) einstellbar ist, wobei sich der Ballon (3) im Innern eines porösen Schlauches (1) befindet, **dadurch gekennzeichnet, dass** der Schlauch (1) als Band ausgebildet ist, das den Ballon (3) an zwei Seiten überragt.

2. Vorrichtung nach Anspruch 1, **dadurch gekennzeichnet, dass** der Schlauch ein textiler Schlauch (1) ist.

3. Vorrichtung nach Anspruch 2, **dadurch gekennzeichnet, dass** der textile Schlauch (1) netzartig ausgebildet ist.

4. Vorrichtung nach einem der vorhergehenden Ansprüche, **dadurch gekennzeichnet, dass** der Schlauch (1) aus einer Maschenware besteht, insbesondere gewirkt oder gestrickt ist.

5. Vorrichtung nach einem der Ansprüche 1 bis 4, **dadurch gekennzeichnet, dass** der Schlauch ein Schlauchgeflecht ist.

6. Vorrichtung nach einem der vorhergehenden Ansprüche, **dadurch gekennzeichnet, dass** der Schlauch (1) zumindest im Bereich (2) des Ballons (3) im Durchmesser elastisch aufweitbar ist.

7. Vorrichtung nach einem der vorhergehenden Ansprüche, **dadurch gekennzeichnet, dass** der Schlauch (1) im Bereich (2) des Ballons (3) einen größeren Durchmesser aufweist als in Bereichen (4) außerhalb des Ballons (3).

8. Vorrichtung nach einem der vorhergehenden Ansprüche, **dadurch gekennzeichnet, dass** der Ballon (3) im Schlauch (1) gegen eine Verschiebung in Längsrichtung des Schlauches fixiert ist.

9. Vorrichtung nach einem der vorhergehenden Ansprüche, **dadurch gekennzeichnet, dass** der Schlauch (1) im Bereich des Ballons (3) kleinporiger ist als außerhalb dieses Bereichs.

10. Vorrichtung nach einem der vorhergehenden Ansprüche, **dadurch gekennzeichnet, dass** der textile Schlauch (1) aus PVDF und/oder Polypropylen besteht.

11. Vorrichtung nach einem der vorhergehenden Ansprüche, **dadurch gekennzeichnet, dass** der Schlauch (1) aus monofilen Fäden gefertigt ist.

12. Vorrichtung nach einem der vorhergehenden Ansprüche, **dadurch gekennzeichnet, dass** der Ballon (3) elastisch aufweitbar ist.

13. Vorrichtung nach einem der vorhergehenden Ansprüche, **dadurch gekennzeichnet, dass** der Ballon (3) und vorzugsweise auch die mindestens eine Fluidleitung (6) aus Silikon bestehen.

14. Vorrichtung nach einem der vorhergehenden Ansprüche, **dadurch gekennzeichnet, dass** mindestens eine Fluidleitung (6) entlang des Schlauches (1) in dessen Inneren mindestens bis zum Schlauchende geführt ist.

15. Vorrichtung nach einem der vorhergehenden Ansprüche, **dadurch gekennzeichnet, dass** mindestens eine Fluidleitung (6) zwischen dem Ballon (3) und einem Schlauchende radial aus dem Schlauch (1) durch dessen Wandung herausgeführt ist.

16. Vorrichtung nach einem der vorhergehenden Ansprüche, **dadurch gekennzeichnet, dass** ein freies Ende der Fluidleitung (6) mit einem Fluidventil (7) verschlossen ist.

17. Vorrichtung nach einem der vorhergehenden Ansprüche, **dadurch gekennzeichnet, dass** der Schlauch (11, 21), insbesondere im Bereich des Ballons (13, 23), mit einem Kleber versehen ist.

## Claims

1. Device for the prevention of incontinence, especially urinary incontinence, with an expandable balloon (3) that is connected to at least one fluid conduit (6) by means of which the volume of the balloon (3) can be adjusted, the balloon (3) being located inside a porous tube (1), **characterized in that** the tube (1) is designed as a band which protrudes beyond the balloon (3) at both ends.

2. Device according to Claim 1, **characterized in that** the tube is a textile tube (1).

3. Device according to Claim 2, **characterized in that** the textile tube (1) has a net-like design.

4. Device according to one of the preceding claims, **characterized in that** the tube (1) is made from a looped fabric, in particular a formed-loop knit or drawn-loop knit.

5. Device according to one of Claims 1 through 4, **characterized in that** the tube is a tubular braid.

6. Device according to one of the preceding claims, **characterized in that** the tube (1), at least in the area (2) of the balloon (3), is elastically expandable in diameter.

7. Device according to one of the preceding claims, **characterized in that** the tube (1) has a greater diameter in the area (2) of the balloon (3) than it does in the areas (4) outside the balloon (3).

8. Device according to one of the preceding claims, **characterized in that** the balloon (3) is fixed in the tube (1) against displacement in the longitudinal direction of the tube.

9. Device according to one of the preceding claims, **characterized in that** the tube (1) has smaller pores in the area of the balloon (3) than it does outside this area.

10. Device according to one of the preceding claims, **characterized in that** the textile tube (1) is made of PVDF and/or polypropylene.

11. Device according to one of the preceding claims, **characterized in that** the tube (1) is produced from monofilament threads.

12. Device according to one of the preceding claims, **characterized in that** the balloon (3) is elastically expandable.

13. Device according to one of the preceding claims, **characterized in that** the balloon (3) and preferably also the at least one fluid conduit (6) are made of silicone.

14. Device according to one of the preceding claims, **characterized in that** at least one fluid conduit (6) is routed along the inside of the tube (1), at least as far as the end of the tube.

15. Device according to one of the preceding claims, **characterized in that** at least one fluid conduit (6) between the balloon (3) and an end of the tube is routed radially out of the tube (1) through the wall of the latter.

16. Device according to one of the preceding claims, **characterized in that** a free end of the fluid conduit (6) is closed with a fluid valve (7).

17. Device according to one of the preceding claims, **characterized in that** the tube (11, 21), particularly in the area of the balloon (13, 23), is provided with an adhesive.

## Revendications

1. Dispositif permettant d'éviter l'incontinence, en particulier l'incontinence urinaire, avec un ballon expansible (3), qui est raccordé à au moins une conduite de fluide (6), au moyen de laquelle le volume du ballon (3) est réglable, dans lequel le ballon (3) se trouve à l'intérieur d'un tuyau poreux (1), **caractérisé en ce que** le tuyau (1) est réalisé sous la forme d'une bande, qui dépasse le ballon (3) sur deux côtés.

2. Dispositif selon la revendication 1, **caractérisé en ce que** le tuyau est un tuyau textile (1).

3. Dispositif selon la revendication 2, **caractérisé en ce que** le tuyau textile (1) est réalisé en forme de filet.

4. Dispositif selon l'une quelconque des revendications précédentes, **caractérisé en ce que** le tuyau (1) se compose d'un tissu à mailles, en particulier maillé ou tricoté.

5. Dispositif selon l'une quelconque des revendications 1 à 4, **caractérisé en ce que** le tuyau est un tuyau tressé.

6. Dispositif selon l'une quelconque des revendications précédentes, **caractérisé en ce que** le
tuyau (1) est extensible élastiquement en diamètre au moins dans la région (2) du ballon (3).

7. Dispositif selon l'une quelconque des revendications précédentes, **caractérisé en ce que** le tuyau (1) présente un plus grand diamètre dans la région (2) du ballon (3) que dans des régions (4) à l'extérieur du ballon (3).

8. Dispositif selon l'une quelconque des revendications précédentes, **caractérisé en ce que** le ballon (3) est fixé dans le tuyau (1) contre un déplacement dans la direction longitudinale du tuyau.

9. Dispositif selon l'une quelconque des revendications précédentes, **caractérisé en ce que** le tuyau (1) présente des pores plus petits dans la région du ballon (3) qu'à l'extérieur de cette région.

10. Dispositif selon l'une quelconque des revendications précédentes, **caractérisé en ce que** le tuyau textile (1) se compose de PVDF et/ou de polypropylène.

11. Dispositif selon l'une quelconque des revendications précédentes, **caractérisé en ce que** le tuyau (1) est fabriqué à partir de fibres unifilaires.

12. Dispositif selon l'une quelconque des revendications précédentes, **caractérisé en ce que** le ballon (3) est expansible élastiquement.

13. Dispositif selon l'une quelconque des revendications précédentes, **caractérisé en ce que** le ballon (3) et de préférence aussi ladite au moins une conduite de fluide (6) se composent de silicone.

14. Dispositif selon l'une quelconque des revendications précédentes, **caractérisé en ce qu'**au moins une conduite de fluide (6) est menée le long du tuyau (1) à l'intérieur de celui-ci au moins jusqu'à l'extrémité du tuyau.

15. Dispositif selon l'une quelconque des revendications précédentes, **caractérisé en ce qu'**au moins une conduite de fluide (6) est menée radialement hors du tuyau (1), à travers la paroi de celui-ci, entre le ballon (3) et une extrémité de tuyau.

16. Dispositif selon l'une quelconque des revendications précédentes, **caractérisé en ce qu'**une extrémité libre de la conduite de fluide (6) est fermée avec une soupape à fluide (7).

17. Dispositif selon l'une quelconque des revendications précédentes, **caractérisé en ce que** le tuyau (11, 21) est muni d'un adhésif, en particulier dans la région du ballon (13, 23).
